Europäisches Patentamt

European Patent Office

Office européen des brevets

(19)

(11) **EP 1 183 323 B1**

(12) **EUROPÄISCHE PATENTSCHRIFT**

(45) Veröffentlichungstag und Bekanntmachung des Hinweises auf die Patenterteilung:
**21.04.2004 Patentblatt 2004/17**

(21) Anmeldenummer: **00936748.3**

(22) Anmeldetag: **17.05.2000**

(51) Int Cl.⁷: **C11B 3/10**, C11B 1/10

(86) Internationale Anmeldenummer:
**PCT/EP2000/004469**

(87) Internationale Veröffentlichungsnummer:
**WO 2000/071650 (30.11.2000 Gazette 2000/48)**

(54) **VERFAHREN ZUR AUFREINIGUNG VON NATURÖLEN, DAFÜR GEEIGNETE VORRICHTUNG UND VERWENDUNG DER PRODUKTE**

METHOD FOR PURIFYING NATURAL OILS, DEVICE SUITABLE THEREFOR AND USE OF SAID PRODUCTS

PROCEDE DE PURIFICATION D'HUILES NATURELLES, DISPOSITIF Y RELATIF ET UTILISATION DES PRODUITS OBTENUS

(84) Benannte Vertragsstaaten:
**AT BE CH DE FR GB IT LI LU NL**

(30) Priorität: **21.05.1999 DE 19923558**

(43) Veröffentlichungstag der Anmeldung:
**06.03.2002 Patentblatt 2002/10**

(73) Patentinhaber: **KD Pharma Bexbach GmbH**
**66450 Bexbach (DE)**

(72) Erfinder:
• **KRUMBHOLZ, Rudolf**
**F-57510 Holving (FR)**
• **ENGELHARDT, Heinz**
**D-66129 Bübingen (DE)**
• **SCHIRRA, Norbert**
**D-66333 Völklingen (DE)**
• **TREITZ, Manfred**
**D-66352 Grossrosseln (DE)**

(74) Vertreter: **Ackermann, Joachim, Dr. et al**
**Postfach 11 13 26**
**60048 Frankfurt am Main (DE)**

(56) Entgegenhaltungen:
**GB-A- 2 246 362      US-A- 5 414 100**
**US-A- 5 719 302**

• **YOSHITSUGU KOSUGI ET AL: "SYNTHESIS OF TRIACYLGLYCEROL FROM POLYUNSATURATED FATTY ACID BY IMMOBILIZED LIPASE" JOURNAL OF THE AMERICAN OIL CHEMISTS' SOCIETY,US,AMERICAN OIL CHEMISTS' SOCIETY. CHAMPAIGN, Bd. 71, Nr. 12, 1. Dezember 1994 (1994-12-01), Seiten 1397-1403, XP000480794 ISSN: 0003-021X**

## Beschreibung

Die Erfindung betrifft ein Verfahren zur Aufreinigung von Naturölen enthaltend höhere ungesättigte Fettsäuren und die Verwendung der gereinigten Produkte in Lebensmitteln und in der Pharmazie.

Das Interesse der Industrie an neuen Verfahren zur Gewinnung von physiologisch wichtigen Fettsäuren aus Naturölen, insbesondere von reinen höheren $\omega$-3-und $\omega$-6 Fettsäuren, ist groß.

Der therapeutische Effekte von $\omega$-3-und $\omega$-6 Fettsäuren, wie z. B. Stearidonsäure (SDA), Eicosapentaensäure (EPA), Docosahexaensäure (DHA) oder deren Estern, wie den Ethylestem, zur Behandlung von Artherosklerose, rheumatischen Beschwerden, Morbus Crohn ist bekannt. Zur Erzielung eines therapeutischen Effektes - einschließlich eines präventiven und kurativen Effektes - werden tägliche Dosen von etwa 1 - 2 g an reinen höheren ungesättigten Fettsäuren benötigt (Vgl. P. Grimm, Ärztezeitschrift für Naturheilverfahren 35, 3 (1994), S. 202 u.v.a.).

Die Gewinnung von ungesättigten Fettsäuren kann aus nativen Quellen, wie Naturölen, erfolgen. Besonders Fischöl weist einen hohen Gehalt an höheren ungesättigten Fettsäuren auf. Weiterhin ist ein hoher Gehalt an ungesättigten Fettsäuren in Algen (Vgl. B.W. Nichols et al., Phytochemistry, Vol. 88, pp. 1907 ff. (1969); G. Ahlgren et al., J. Phycol. 28, 37-50 (1992); A. Seto et al., JAOCS, Vol. 61, no. 5 (1984) S. 892 ff.) beschrieben. Zumeist sind jedoch solche Naturöle von eigentümlichem Geschmack und Geruch, welche durch Stoffe verursacht werden können, die in geringen Mengen vorliegen können (teilweise ppm Bereich).

Gerade der Einsatz von höheren $\omega$-3-und $\omega$-6 Fettsäuren in Lebensmitteln und Pharmazie erfordert daher zur Erzielung einer hohen Akzeptanz beim Konsumenten bzw. Patienten die Verringerung oder die Beseitigung von Fremd- und Begleitstoffen, die für den Geruch und Geschmack verantwortlich sind (z.B. in Fischöl Amine etc.).

Bekannt ist die Verkapselung und die Verwendung des erhaltenen Pulvers in Backund Teigwaren (H. Nielsen, J. Sci. Food Agric., 1992, 59, 559-562).

Dieses Verfahren ist jedoch aufwendig und daher kostspielig. Zudem ist von Nachteil, daß das derart erhaltene Pulver z.B. in Flüssigkeiten (Getränke etc.) aufgrund unerwünschter Trübung und schlechter Sensorik nur bedingt oder gar nicht einsetzbar ist.

Es ist daher wünschenswert die im Naturöl enthaltenden Fettsäuren derart bereitzustellen, daß diese direkt oder in einer verkapselten Form als Feststoff oder als Emulsion in Pharmazie und Lebensmitteln eingesetzt werden können, ohne dass die Nachteile vorbekannter Produkte zum Tragen kommen.

Im Stand der Technik werden derartige Naturöle oder deren Auszüge zur Reduzierung des Geruches entweder unter Vakuum erhitzt, um die leichtflüchtigen Stoffe zu entfemen oder einer sogenannten Bleichung unterzogen.

Hierzu werden sogenannte Bleicherden (Fullersche Erde, Aluminiumsilikate) zu 1-3 Gew.-% dem Fischöl zugesetzt und für 2 Stunden auf ca. 60°C erhitzt. Anschließend wird die Bleicherde durch Filtration entfernt (Vgl. F. D. Gunstone et al. (Ed.), The Lipid Handbook, London, (1994), S. 287 ff).

In der JP-A-10/338,621 wird ein Verfahren zur Herstellung eines Aerosols beschrieben, worin u.a. mittels MgO-Partikeln auf einer Silica-Oberfläche Gerüche aus der Luft entfernt werden, die durch leichtflüchtige Fettsäuren entstehen.

In der WO-A-98/13,138 werden Siliciumoxide mit einem geringen Anteil an leichtflüchtigen Stoffen im basischen pH beschrieben, die den Geruch von organischen Harzen vermindern. Vor allem der durch Fettsäureamide resultierende Geruch wird verringert.

In der CZ-A-278,992 wird ein Verfahren zur Erzielung eines geruchsfreien Pflanzenöls nach der Hydrierung beschrieben. Dabei werden 500 g hydriertes Pflanzenöl mit einer Mischung von 9g Bleicherde und 1g Aktivkohle 15 min. gerührt.

In der WO-A-93/10,207 wird Fischöl mit einem milden Fischgeruch beschrieben. Dabei wird Fischöl (18%EPA, 12% DHA) mit Trisylsilica für 1 Stunde im Vakuum auf 80°C erhitzt. Die Mischung wird auf 40°C abgekühlt, Hexan wird hinzugefügt und diese Mischung wird durch ein Kieselgelbett filtriert. Das Filtrat wird destilliert, um das Hexan zu entfernen.

In der JP-A-01/272,510 wird ein Verfahren zur Herstellung eines geruchsfreien Lanolinfettsäureesters beschrieben. Dazu wird der Lanolinfettsäureester in einem unpolaren Lösemittel (Hexan) gelöst und durch eine mit Silica gepackte Säule gegeben und das Lösemittel abdestilliert.

In der EP-A-298,293 wird zur Entfernung von unerwünschten Nebenprodukten aus Fischöl, das Fischöl in Hexan gelöst und über eine Kieselgelsäule gepumpt. Dabei werden nichtverseifbare Anteile, oxidierte Triglyceride etc, reduziert. Über den Geruch nach der Behandlung wird keine Aussage gemacht.

Die JP-A-62/181,398 beschreibt die Herstellung eines geruchsfreien Marinöls. Das Marinöl wird in Hexan gelöst und zusammen mit Silicateilchen für 2 Stunden bei 30°C erhitzt. Der Überstand wird abdestilliert und es wird ein Öl mit hohen Gehalten an EPA und DHA, die frei von Geruch sind, erhalten.

Die JP-A-60/043,119 beschreibt ebenfalls die Herstellung eines Öls frei von Fischgeruch. Dazu wird das Öl mit Streptococcus lactis vorbehandelt und diese Vormischung wird mit 4 bis 10 Teilen Öl für 60 min bei 35°C gerührt. Die festen Bestandteile werden entfernt, es wird neutralisiert und die Mischung mit 0,2% Aktivkohle und 2% Bleicherde bei Vakuum 30 min entfärbt.

In der DE-A-2,806,706 wird ein Pflanzenöl mit akzeptablem Geruch hergestellt. Nach Hydrierung und Säurebehandlung wird durch ein Bett von granulierter Aktivkohle gepumpt. Ziel ist die Reduzierung des Phosphor-Gehaltes. Anschließend erfolgt Desodorierung durch Erhitzen im Vakuum.

In der DE-A-2,622,520 wird eine Apparatur zur kontinuierlichen Vorreinigung und Bleichung von Ölen beschrieben. Im Prinzip werden Phosphate, Wasser, Lösemittel, freie Säuren und der Geruch durch Bleicherde entfernt.

In der US-A-3,649,656 werden zur Verbesserung der Farbe und des Geruches dem Naturöl Additive aus Lignin, Calciumsilikate und/oder Magnesiumsilikate (0,05 Gew.-%) beigemengt.

In der DE-A-19638459 wird ein Verfahren zur Reinigung von Fetten und Ölen tierischen und vegetarischen Ursprungs beschrieben. Dabei werden freie Fettsäuren, Geruchs- und Geschmacksstoffe mit Polyethylenglykol extrahiert.

In allen bekannten Verfahren ist von Nachteil, daß entweder das Naturöl mit Aktivkohle oder Bleicherden unter Qualitätseinbuße thermisch belastet wird oder gar in einem unpolaren Lösungsmittel - wie z.B. Hexan - gelöst wird. Zumeist erfolgt eine Lösungsmittelextraktion. Dabei kann das Lösemittel nur mit hohem Aufwand praktisch vollständig entfernt werden.

Zudem weisen die im Handel erhältlichen aufgearbeiteten Öle üblicherweise Eigengeruch und Eigengeschmack auf. Lediglich die Intensität schwankt von Verfahren zu Verfahren.

Daher hat die Erfindung zur Aufgabe ein Verfahren zur Gewinnung eines geschmacks- und geruchsfreien Öls enthaltend höhere ungesättigte Fettsäuren, vorzugsweise ω-3-und ω-6 Fettsäuren, aus Naturölen bereitzustellen.

Unter höheren ungesättigten Fettsäuren werden insbesondere solche Fettsäuren verstanden, die drei oder mehr Doppelbindungen enthalten.

Die Aufgabe wird in überraschender Weise dadurch gelöst, daß das Naturöl über Aluminiumoxid als Adsorbens geführt wird (nachstehend erfindungsgemäßes Verfahren).

Daher betrifft das erfindungsgemäße Verfahren ebenfalls eine adsorptive Filtration und/oder eine lösemittelfreie Chromatographie von Naturölen an Aluminiumoxid. Des weiteren kann das erfindungsgemäße Verfahren im weitesten übergeordneten Sinne als ein Reinigungsverfahren zur Beseitigung unerwünschter Fremd- und/oder Begleitstoffe, wie Geschmacks- und Geruchsstoffen, aus Naturölen verstanden werden.

Als geeignetes Adsorbens zur Beseitigung unerwünschter Fremd- und/oder Begleitstoffe des Naturöls erweist sich Aluminiumoxid mit einer Partikelgröße von 1-1000 μm, vorzugsweise 50 - 200 μm, welches aber nicht notwendiger Weise gemörsert, mit Säure und Base gewaschen oder angeätzt wird.

Da im erfindungsgemäßen Verfahren kein Lösungsmittel verwendet werden muß, ist die Biokompatibilität der geruch- und geschmackfreien Verfahrensprodukte gewährleistet, wodurch die Verwendung der Verfahrensprodukte in Pharmazie und als Lebensmittelzusatzstoff besonders geeignet ist.

Naturöle im Sinne dieser Erfindung sind solche aus nativen Quellen, mit einem natürlichen hohen Gehalt an ungesättigten Fettsäuren, insbesondere ω-3-und ω-6 Fettsäuren - mehrfach ungesättigt und mit mehr als 16 C-Kohlenstoffatomen, wie EPA, DHA, DPA, Stearidonsäure 18:4, Arachidonsäure 20:4 und alpha- und gamma-Linolensäure 18:3.

Besonders bevorzugt als Ausgangsöl für das erfindungsgemäße Verfahren ist Fischöl. Ebenfalls einsetzbar sind Algenöle, Öle aus Pilze, Öle aus Mikroorganismen und Pflanzenöle. Zur allgemeinen Herstellung solcher Naturöle wird auf Gunstone supra S. 167 ff. und 253 ff.; H. Franke et al., 1st European Workshop on Microalgea Biotechnology; Bergholz-Rehbrücke, June 10-12, 1992, S. 41 ff; C. Ratledge et al. (Ed), Microbial Lipids, London (1994) S. 394 ff verwiesen.

In einer bevorzugten Ausführungsform wird das Naturöl mit Hilfe chromatographischer Verfahren fraktioniert (Vor-Fraktionierung) und Fraktionen enthaltend höhere Fettsäuren werden angereichert und dem erfindungsgemäßen Verfahren als Ausgangsöl zugeführt.

Bevorzugte chromatographische Verfahren sind solche der schonenden überkritischen Fluidchromatographie wie Chromatographie mit überkritischen Fluiden (SFC), beispielsweise mit Kohlendioxid, Extraktion mit überkritischen Fluiden (SFE) oder Gegenstrom SFE (cc-SFE), da eben solche besonders die Biokompatibilität der Produkte gewährleisten. Ebenso zur Fraktionierung von Naturölen geeignet ist insbesondere die HPLC sowie andere dem Durchschnittsfachmann bekannte Verfahren der Fraktionierung.

Die in den Naturölen enthaltenden Fettsäuren werden in Lipiden und Lipoiden nach bekannten Verfahren umgeestert (Vgl. N. Nagle et al., Applied Biochemistry and Biotechnology, Vol. 24/25, 1990, S. 355 ff; M. Piorreck et al. , Phytoochemistry, Vol. 23, No. 2, S. 207 ff, 1984, W.W. Christie; Gas Chromatography and Lipids, Oily Press, 1989, S. 64; Gunstone supra S. 394). Es werden Fettsäurealkylester, vorzugsweise Fettsäureethylester erhalten, welche über Fraktionierung oder direkt dem erfindungsgemäßen Verfahren zugeführt werden.

Zur ausführung der Erfindung ist eine Vorrichtung oder eine funktionelle Anordnung bestehend mindestens aus einem Vorratsreservoir welches über eine Pumpe mit einer Säule enthaltend Aluminiumoxid als Adsorbens verbunden ist mit anschließender Fraktionierung geeignet.

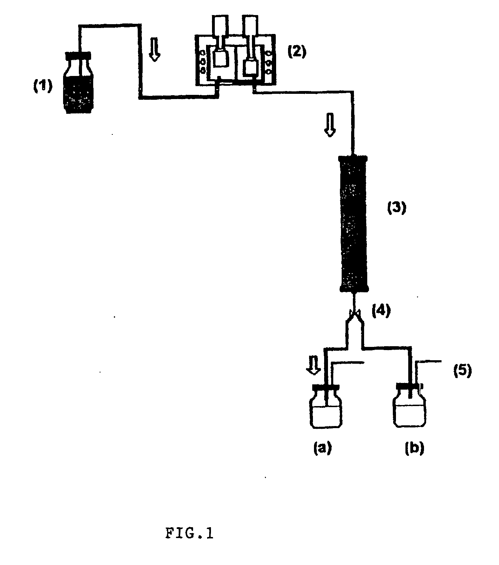

Eine Ausführungsform der Vorrichtung wird in Figur 1 dargestellt. Darin bedeuten (1) ein Vorratsgefäß; (2) eine Pumpe; (3) eine Säule mit Adsorbens; (4) ein Umschaltventil; (5) Gefäß(e) zum Auffangen der Eluate.

Zur Durchführung des erfindungsgemäßen Verfahrens mittel der in Figur 1 abgebildeten prinzipiellen funktionellen Anordnung wird Säule (3) (z.B. Länge: 35 cm, i.D. 2,5 cm) mit Aluminiumoxid gefüllt, vorzugsweise Alumina Super I-Qualität (Teilchendurchmesser 63-200 μm) - ohne weitere Vorbehandlung. Mit Hilfe einer Pumpe (2) wird das Ausgangsöl (hier z.B: Fischöl) direkt über die Säule gepumpt. Der von der Pumpe aufzubringende Druck hängt von der Viskosität des Öls und vom Fluß ab. Der Fluß beträgt 0,1 bis 15 Säulenvolumina/h, vorzugsweise jedoch 3 Säulenvolumina/h. Das erhaltene Öl wird über ein Umschaltventil (4) geleitet und in verschiedenen Fraktionen (5)(a), (5)(b) etc. aufgefangen und zwar werden vorzugsweise solche Fraktionen freigegeben mit einer Peroxidzahl von 0 bis 10, besonders bevorzugt kleiner 5, mit einer Säurezahl von 0 bis 2, besonders bevorzugt kleiner 1, mit einem Anisidinwert von 0 bis 30 besonders bevorzugt kleiner 20 (siehe Beispiel 5).

Das erhaltene Öl ist völlig frei von Eigengeruch oder Eigengeschmack des Naturöls und ist nahezu farblos; der Geruch wird von Testpersonen fruchtig und apfelartig oder neutral beschrieben.

Im Verlauf des erfindungsgemäßen Verfahrens erfolgt keine Änderung des Fettsäurespektrums, vielmehr werden die Fremd- und Begleitstoffe irreversibel am Adsorbens retardiert.

In einer weiteren bevorzugten Ausführungsform erfolgt das erfindungsgemäße Verfahren im Rahmen eines chromatographischen Verfahren ohne Lösungsmittel, wie in Figur 1 als Kurzweg-Säulenchromatographie beschrieben und erläutert.

Ganz besonders bevorzugt wird das erfindungsgemäße Verfahren als Radial-Säulenchromatographie mit dünner Schichtdicke durchgeführt. Bevorzugt sind Schichtdicken von 1 bis 50 cm, besonders bevorzugt 3 bis 35 cm und ganz besonders bevorzugt ist eine Schichtdicke von 30 cm. Großtechnisch kann die Radial-Säulenchromatographie beispielsweise im Rahmen eines ARCF-Verfahrens (Adsorptive Radial Coloumn Filtration, Firma Sepragen Emmen, Netherlands) ausgeführt werden.

In einer bevorzugten Ausführungsform können Fettsäureethylester weiter an bestimmten Fettsäuren und/oder Fettsäuregruppen aufkonzentriert werden. Dies kann mittels einer Harnstofffällung erfolgen. Man erhält dann ein Ausgangsöl, daß ca. 45% EPA und ca. 75% omega-3-Fettsäureethylester enthält (Hierbei handelt es sich um typische Werte bei einer Harnstofffällung mit Fischöl).

Beispielsweise werden bei einer SFC Trennung von Fischöl typischerweise folgende Produkte erhalten: SDA mit Gehalten 30-40%, EPA mit Gehalten 85-95%, DHA mit Gehalten von 50-70%, Summe der omega-3-Fettsäuren größer als 90%.

Die biokompatiblen Verfahrungsprodukte können als Lebensmittelzusatzstoffe und in der Pharmazie verwendet werden. Die erhaltenen Öle (Verfahrensprodukte) können entweder direkt oder nach einer weiteren Verarbeitung (Pulver, Emulsion, Derivatisierung) als Lebensmittel und/oder Lebensmittelzusatzstoffe sowie in der Pharmazie eingesetzt werden. Formulierungen zur Applikation können sein solche wie Kapsel, Dragee etc.

Die nachfolgenden Beispiel dienen zur näheren Erläuterung der Erfindung, ohne die Erfindung auf diese Beispiele einzuschränken.

Beispiele:

Beispiel 1:

Fettsäureethylester (Gehalt omega-3-Fettsäureethylester > 90%), das mittels konventioneller Bleichung vorgereinigt wurde, wurde mit dem erfindungsgemäßen Verfahren aufgereinigt.

Es wurden 0,621 kg dieses Produktes über eine Säule mit 0,195 kg $Al_2O_3$ (Schichtdicke ca. 30 cm) gegeben. Man erhielt 0,426 kg Fettsäureethylester. Dies entspricht einer Ausbeute von 68,6%. Dauer: ca. 52 min. Es wurden ca. 0,63 kg Fettsäureethylester gepumpt, Differenzmenge befand sich in der Säule.

Das Verhältnis von eingesetzter Ölmenge (kg) zu eingesetztem $Al_2O_3$ (kg) beträgt 3,2:1.

Beispiel 2:

Fettsäureethylester (Gehalt omega-3-Fettsäureethylester > 90%), das mittels konventioneller Bleichung vorgereinigt wurde, wurde mit dem beanspruchten Verfahren aufgereinigt.

Es wurden 0,567 kg dieses Produktes über eine Säule mit 0,196 kg $Al_2O_3$ (Schichtdicke ca. 30 cm) gegeben. Man erhielt 0,371 kg Fettsäureethylester. Dies entspricht einer Ausbeute von 65,4%. Dauer: ca. 48 min.

Das Verhältnis von eingesetzter Ölmenge (kg) zu eingesetztem $Al_2O_3$ (kg) beträgt 2,9:1.

Beispiel 3:

Ein Fettsäureethylesterprodukt (Gehalt omega-3-Fettsäureethylester > 90%), das mittels SFC gewonnen wurde, wurde mit dem erfindungsgemäßen Verfahren aufgereinigt.

Es wurden 1,812 kg dieses Produktes über eine Säule mit 0,196 kg $Al_2O_3$ (Schichtdicke ca. 30 cm) gegeben. Man erhielt 1,534 kg Fettsäureethylester. Dies entspricht einer Ausbeute von 84,6%. Dauer: ca. 2,5 Stunden.

Das Verhältnis von eingesetzter Ölmenge (kg) zu eingesetztem $Al_2O_3$ (kg) beträgt 9,3:1.

Beispiel 4:

Adsorptive Filtration und / oder Chromatographie mit geringer Schichtdicke

Auf einer Nutsche (Durchmesser 9,3 cm) wurde ein Bett von ca. 3 cm (entspricht Schichtdicke) ca. 216 g Aluminiumoxid aufgeschüttet. 400 g Fischöl (66% DHA) wurden mittels Vakuum durch die Nutsche gesaugt. Man erhielt 311,9 g Fettsäureethylester. Dies entspricht einer Ausbeute von 78,0%.

Beispiel 5.

Bestimmung der Säurezahl, Peroxidzahl, Anisidinwerte

a) Arbeitsvorschrift zur Bestimmung der Peroxidzahl (POZ) (Methode nach Wheeler; aus: DGF-Einheitsmethoden, Deutsche Einheitsmethoden zur Untersuchung von Fetten, Fettprodukten, Tensiden und verwandten Stoffen, Hrsg.: Deutsche Gesellschaft für Fettwissenschaft e.V., Münster, Wissenschaftliche Verlagsgesellschaft mbH Stuttgart, 1989 C-VI 6a (84)); vgl. IUPAC method 2.501 (1985), AOCS method Cd 8-53 (AOCS, 1987) Etwa 10 g Probe wurden auf $\pm$ 0,1 mg genau eingewogen und in 30 ml Eisessig und Chloroform (Verhältnis 3 + 2 Volumenteile) gemischt unter Umschwenken gelöst. Nach Zugabe von 0,5 ml einer gesättigten Kaliumiodid-Lösung (10 g KJ + 10 g $H_2O$) wurde genau 60 s lang kräftig geschüttelt und anschließend im Dunkeln aufbewahrt.

Unmittelbar danach wurde die Lösung mit 30 ml Wasser verdünnt und das ausgeschiedene Iod mit der 0,01 M Natriumthiosulfat-Maßlösung titriert. Die etwa 1 %ige Stärke-Lösung wurde vor Beginn der Titration zugegeben.

In gleicher Weise wurde ein Blindversuch durchgeführt, bei dem nicht mehr als 0,1 ml Maßlösung verbraucht werden sollten.

Auswertung:

$$POZ = \frac{(a-b)*N}{E} * 1000$$

a    Verbrauch der Maßlösung im Hauptversuch in ml
b    Verbrauch der Maßlösung im Blindversuch in ml
N    Titer der verwendeten Maßlösung in mol/l
E    Probeneinwaage in g

b.) Arbeitsvorschrift zur Bestimmung der Säurezahl (SZ) (Stand März 1995) (Vgl. DGF-Einheitsmethoden supra: C-V 2 (81); IUPAC method 2.201 (1985); AOCS method Ca 5a-40 (AOCS, 1978)

Es wurden 5 - 10 g Probe auf $\pm$ 0,1 % genau eingewogen, die Einwaage ist probenspezifisch zu wählen. Zu diesem Zweck wurde von jeder Probe erst nur eine Einwaage (ca. 7,5 g) angesetzt und bei einer "Schnelltitration" der Verbrauch an Maßlösung festgestellt. Sollte der Verbrauch in keinem vernünftigen Bereich liegen, wurden die nächsten Einwaagen in die entsprechende Richtung verändert, d.h. bei extrem hohem Verbrauch wird die Einwaage verringert und umgekehrt.

Die Einwaage wurde in etwa 50 ml LM-Gemisch gelöst (LM-Gemisch: Ethanol (100 Vol.-%ig) und Diethylether (Verhältnis 1 : 1 Volumenteil), welches mit 0,1 mol/l Kaliumhydroxid-Maßlösung gegen Phenolphthalein (1%ig in Ethanol) neutralisiert wird) anschließend mit einigen Tropfen Phenolphthalein-Lösung versetzt und mit Kaliumhydroxid-Maßlösung bis zur bleibenden Rotfärbung titriert.

Auswertung:

$$SZ = \frac{a * N * 56,1}{E}$$

a    Verbrauch an Kaliumhydroxid-Maßlösung in ml

N      Titer der Maßlösung in mol/l
E      Probeneinwaage in g
56,1   Molare Masse von KOH

Bei mineralsäurefreien Proben kann aus der SZ der prozentuale Gehalt an freien Fettsäuren (FFA) wie folgt berechnet werden:

Zur angenäherten Berechnung wird die molare Masse (M) von der Ölsäure (M = 282) zugrundegelegt.
    EPA (M= 302,5)
    DHA (M= 328,5)

$$FFA \ (\%) = SZ * (282/561)$$

c) Bestimmung der Anisidin Zahl:

Die Bestimmung erfolgte nach Europäisches Arznelbuch, Deutscher Apotheker Verlag, Stuttgart; Govi-Verlag-Pharmazeutischer Verlag GmbH, Eschborn, Nachtrag 1998; 1998,1250; S.540ff

Die Bestimmung mußte so schnell wie möglich und unter Ausschluß direkter Lichteinwirkung durchgeführt werden.

Untersuchungslösung a:

0,500 g Probe wurden in Isooctan zu 25,0 ml gelöst.

Untersuchungslösung b:

5,0 ml der Untersuchungslösung a wurden mit 1,0 ml des p-Anisidin Reagenz (2,5g p-Anisidin in 1 l Essigsäure 98%) versetzt, geschüttelt und unter Lichtschutz aufbewahrt.

Referenzlösung:

5,0 ml Isooctan wurden mit 1,0 ml des p-Anisidin Reagenz versetzt, geschüttelt und unter Lichtschutz aufbewahrt.

Messung: UV/VIS Shimadzu UV 160

Die Absorption der Untersuchungslösung a wurde gegen Isooctan als Kompensationsflüssigkeit bei 350 nm gemessen.

Genau 10 min nach der Herstellung von Untersuchungslösung b wurde die Absorption dieser Lösung bei 350 nm gegen die Referenzlösung als Kompensationsflüssigkeit gemessen.

Auswertung:

Die Anisidinzahl wird nach folgender Formel berechnet:

$$25 \times \frac{(1,2 \, A_b - A_a)}{m}$$

Ab = Absorption der Untersuchungslösung b bei 350 nm
Aa = Absorption der Untersuchungslösung a bei 350 nm
m = Probeneinwaage in g für die Untersuchungslösung a

Für die Naturöle gemäß den Beispielen 1 bis 4 ergaben sich bei Durchführung der in den Beispielen gemäß 5 a)-c) beschriebenen Messungen die folgenden Werte:

Tabelle:

| Analysenwerte vor und nach Aufreinigung der beschriebenen Beispiele | | | | | | | | |
|---|---|---|---|---|---|---|---|---|
| Parameter | Bsp.1 Al | Bsp.1 S1 | Bsp.2 Al | Bsp.2 S1 | Bsp.3 Al | Bsp.3 S1 | Bsp.4 Al | Bsp.4 S1 |
| POZ | 0,62 | n.n. | n.n. | 1,6 | 1,1 | 1,5 | 13,1 | 3,3 |
| Anisidin | 39,2 | 0,5 | 58,1 | 1,38 | 5,6 | 0,3 | 2,01 | 1,13 |
| SZ | 0,91 | 0,07 | 2,89 | 0,12 | 0,7 | 0,1 | 0,39 | 0,19 |
| Farbe (430 nm) | 0,21 | 0,1 | 0,22 | 0,002 | 0,02 | 0,002 | 0,016 | 0,00 |
| Geschmack | ++ | - | ++ | - | + | - | ++ | - |
| Geruch | ++ | - | ++ | - | + | - | ++ | - |

++: deutlicher Fischgeruch oder deutlicher Fischgeschmack

+: leichter Fischgeruch oder leichter Fischgeschmack

-: kein Fischgeruch oder kein Fischgeschmack

n.n.: nicht nachweisbar        POZ: Peroxidzahl

Al: Ausgangsöl        Anisidin: p-Anisidinwert

S1: aufgereinigtes Öl        SZ: Säurezahl

Wie aus der Tabelle zu entnehmen ist werden auch freie Säuren entfernt und der Anisidinwert und Peroxidzahl gesenkt. Der gemessene Peroxidwert hängt bei kleinen Werten (nahe 1) von der Effektivität des Fraktionierungssystems ab, d.h. wie effektiv das Eindringen von Luft verhindert werden kann.

Nach Beendigung des Verfahrens kann das auf dem Säulenmaterial verbliebene Öl mit einem Lösemittel, wie z.B. Hexan, aber vorzugsweise mit überkritischem oder flüssigem $CO_2$ von der Säule eluiert werden. Dieses Öl kann erneut zur Aufreinigung benutzt werden.

Polare Lösemittel wie z.B. Ethanol sind weniger geeignet, da teilweise adsorbierte Stoffe wieder eluiert werden können.

Im Text verwendete Abkürzungen:

DHA        Docosahexaensäure (22:6)
EPA        Eicosapentaensäureethylester (20:5)
SDA        Stearidonsäureethylester (18:4)
FAEE        Fettsäureethylester
SFE        Extraktion mit überkritischen Fluiden
SFC        Chromatographie mit überkritischen Fluiden
cc-SFE        Gegenstrom-SFE

**Patentansprüche**

1.    Verfahren zum Aufreinigen von Naturöl enthaltend ungesättigte höhere Fettsäuren durch Verringerung oder Beseitigung von geruchs- und/oder geschmacksverursachenden Fremd- und/oder Begleitstoffen aus dem Naturöl, wobei die im Naturöl enthaltenen Fettsäuren zu Fettsäurealkylestern umgeestert werden und ohne den Einsatz von Lösungsmitteln als adsorptive Filtration und / oder als lösungsmittelfreie Chromatographie über Aluminiumoxid als Adsorbens geführt werden.

2.    Verfahren nach Anspruch 1, **dadurch gekennzeichnet, dass** das Naturöl Fischöl, Algenöl, Öle aus Pilzen, Öle aus Mikroorganismen oder Pflanzenöl ist.

3.    Verfahren nach Anspruch 1, **dadurch gekennzeichnet, dass** die Umesterung zu Fettsäureethylestern erfolgt.

4.    Verfahren nach einem der Ansprüche 1 bis 3, **dadurch gekennzeichnet, dass** die Reinigung als Radial-Säulenchromatographie erfolgt.

5.    Verfahren nach einem der Ansprüche 1 bis 4, **dadurch gekennzeichnet, dass** eine Vor-Fraktionierung erfolgt.

**6.** Verfahren nach Anspruch 5, **dadurch gekennzeichnet, dass** die Vorfraktionierung mit Hilfe eines chromatographischen Verfahrens, vorzugsweise mittels Chromatographie mit überkritischen Fluiden (SFC) oder mittels Extraktion mit überkritischen Fluiden (SFE), erfolgt.

**7.** Verfahren nach einem der Ansprüche 1 bis 6, **dadurch gekennzeichnet, dass** der Fluss 0,1 bis 15 Säulenvolumina/h beträgt, vorzugsweise 3 Säulenvolumina/h.

**8.** Verfahren nach einem der Ansprüche 1 bis 7, **dadurch gekennzeichnet, dass** die Partikelgröße des Aluminiumoxids 1 - 1000 μm, vorzugsweise 50 - 200 μm, beträgt und das Aluminiumoxid gegebenenfalls gemörsert, und/oder mit Säure und Base gewaschen und/oder angeätzt wird.

**9.** Verfahren nach einem der Ansprüche 1 bis 8, **dadurch gekennzeichnet, dass** die Schichtdicke 1 bis 50 cm beträgt, vorzugsweise 3 bis 35 cm, besonders bevorzugt 30 cm beträgt.

**10.** Verfahren nach einem der Ansprüche 1 bis 9, **dadurch gekennzeichnet, dass** der Anisidinwert von 0 bis 30, besonders bevorzugt kleiner 20 beträgt.

**11.** Verfahren nach einem der Ansprüche 1 bis 10, **dadurch gekennzeichnet, dass** die Säurezahl von 0 bis 2, besonders bevorzugt kleiner 1 beträgt.

**12.** Verfahren nach einem der Ansprüche 1 bis 11, **dadurch gekennzeichnet, dass** die Peroxidzahl von 0 bis 10, besonders bevorzugt kleiner 5 beträgt.

**Claims**

**1.** Method for purifying natural oil containing unsaturated higher fatty acids by decreasing or eliminating from the natural oil foreign matter and/or accompanying matter causing odour and/or taste, the fatty acids present in the natural oil being transesterified to fatty acid alkyl esters and, without the use of solvents, being passed over alumina as adsorbent as filtration by adsorption and/or as solvent-free chromatography.

**2.** Method according to Claim 1, **characterized in that** the natural oil is fish oil, algal oil, oil from fungi, oil from microorganisms, or vegetable oil.

**3.** Method according to Claim 1, **characterized in that** the transesterification is performed to give fatty acid ethyl esters.

**4.** Method according to one of Claims 1 to 3, **characterized in that** the purification is performed as radial column chromatography.

**5.** Method according to one of Claims 1 to 4, **characterized in that** a prefractionation is performed.

**6.** Method according to Claim 5, **characterized in that** the prefractionation is performed using a chromatographic method, preferably by means of supercritical fluid chromatography (SFC) or using supercritical fluid extraction (SFE).

**7.** Method according to one of Claims 1 to 6, **characterized in that** the flow rate is 0.1 to 15 column volumes/h, preferably 3 column volumes/h.

**8.** Method according to one of Claims 1 to 7, **characterized in that** the particle size of the alumina is 1-1000 μm, preferably 50-200 μm, and the alumina is, if appropriate, ground in a mortar, and/or washed with acid and base and/or etched.

**9.** Method according to one of Claims 1 to 8, **characterized in that** the layer thickness is 1 to 50 cm, preferably 3 to 35 cm, particularly preferably 30 cm.

**10.** Method according to one of Claims 1 to 9, **characterized in that** the anisidine value is from 0 to 30, particularly preferably less than 20.

**11.** Method according to one of Claims 1 to 10, **characterized in that** the acid value is from 0 to 2, particularly preferably less than 1.

**12.** Method according to one of Claims 1 to 11, **characterized in that** the peroxide value is from 0 to 10, particularly preferably less than 5.

**Revendications**

**1.** Procédé de purification d'une huile naturelle contenant des acides gras supérieurs insaturés, par diminution ou élimination, à partir de l'huile naturelle, des substances étrangères et/ou des impuretés qui sont à l'origine d'un goût et/ou d'une odeur, procédé dans lequel les acides gras contenus dans l'huile naturelle sont transestérifiés pour donner des esters alkyliques d'acides gras, et, sans utilisation de solvants, par filtration par absorption et/ou chromatographie sans solvant, sont envoyés sur de l'oxyde d'aluminium servant d'adsorbant.

**2.** Procédé selon la revendication 1, **caractérisé en ce que** l'huile naturelle est une huile de poisson, une huile d'algues, des huiles provenant de champignons, des huiles provenant de microorganismes ou une huile végétale.

**3.** Procédé selon la revendication 1, **caractérisé en ce que** la transestérification conduit à des esters éthyliques d'acides gras.

**4.** Procédé selon l'une des revendications 1 à 3, **caractérisé en ce que** la purification est réalisée sous forme d'une chromatographie radiale sur colonne.

**5.** Procédé selon l'une des revendications 1 à 4, **caractérisé en ce qu'**il comporte un préfractionnement.

**6.** Procédé selon la revendication 5, **caractérisé en ce que** le préfractionnement a lieu par un procédé chromatographique, de préférence par chromatographie en phase supercritique (SFC) ou par extraction en phase supercritique (SFE).

**7.** Procédé selon l'une des revendications 1 à 6, **caractérisé en ce que** le débit est de 0,1 à 15 volumes de colonne/h, de préférence de 3 volumes de colonne/h.

**8.** Procédé selon l'une des revendications 1 à 7, **caractérisé en ce que** la granulométrie de l'oxyde d'aluminium est de 1 à 1000 μm, de préférence de 50 à 200 μm, et que l'oxyde d'aluminium est éventuellement broyé au mortier, et/ou lavé avec un acide et une base, et/ou décapé.

**9.** Procédé selon l'une des revendications 1 à 8, **caractérisé en ce que** l'épaisseur de la couche est de 1 à 50 cm, de préférence de 3 à 35 cm, d'une manière particulièrement préférée de 30 cm.

**10.** Procédé selon l'une des revendications 1 à 9, **caractérisé en ce que** l'indice d'anisidine est de 0 à 30, d'une manière particulièrement préférée inférieur à 20.

**11.** Procédé selon l'une des revendications 1 à 10, **caractérisé en ce que** l'indice d'acide est de 0 à 2, d'une manière particulièrement préférée inférieur à 1.

**12.** Procédé selon l'une des revendications 1 à 11, **caractérisé en ce que** l'indice de peroxyde est de 0 à 10, d'une manière particulièrement préférée inférieur à 5.

(1) (2) (3) (4) (5) (a) (b)

FIG.1